# EUROPÄISCHE PATENTSCHRIFT

(11) **EP 3 814 028 B1**
(45) Veröffentlichungstag und Bekanntmachung des Hinweises auf die Patenterteilung: **02.09.2026**
(21) Anmeldenummer: 19708412.2
(22) Anmeldetag: 20.02.2019
(51) Int. Cl.: B08B 3/08, B08B 9/00, B08B 9/20, A61L 2/10, A47L 15/42, A61L 2/208

(54) **BEHÄLTERBEHANDLUNGSMASCHINE UND VERFAHREN ZUM REINIGEN EINER BEHÄLTERBEHANDLUNGSMASCHINE**
CONTAINER-PROCESSING MACHINE AND METHOD FOR CLEANING A CONTAINER-PROCESSING MACHINE
MACHINE DE TRAITEMENT DE CONTENANT ET PROCÉDÉ DE NETTOYAGE D'UNE MACHINE DE TRAITEMENT DE CONTENANT

(30) Priorität: 26.06.2018 DE 102018210369
(43) Veröffentlichungstag der Anmeldung: 05.05.2021
(73) Patentinhaber: Krones AG, 93073 Neutraubling (DE)
(72) Erfinder: SACHT, Merret, 93073 Neutraubling (DE); DAWARTZ, Matthias, 93073 Neutraubling (DE)
(74) Vertreter: Grünecker Patent- und Rechtsanwälte PartG mbB
(86) Internationale Anmeldenummer: PCT/EP2019/054158
(87) Internationale Veröffentlichungsnummer: WO 2020/001817

(56) Entgegenhaltungen:
- WO-A1-2005/063109
- WO-A1-2008/040316
- DE-A1- 102004 039 084
- DE-A1- 102008 039 674
- DE-U1- 202008 004 775
- JP-A- 2001 258 811
- JP-A- 2006 280 518
- KR-A- 20150 032 037
- HOIGNÉ J. ET AL: "Ozonation of Water: Role of Hydroxyl Radicals as Oxidizing Intermediates", SCIENCE, vol. 190, no. 4216, 21 November 1975 (1975-11-21), US, pages 782 - 784, XP93038324, ISSN: 0036-8075, Retrieved from the Internet <URL:http://dx.doi.org/10.1126/science.190.4216.782> [retrieved on 20230411], DOI: 10.1126/science.190.4216.782

## Beschreibung

Die Erfindung betrifft ein Verfahren gemäß Oberbegriff des Anspruchs 1 sowie eine Behälterbehandlungsmaschine gemäß Oberbegriff des Anspruchs 9.

Mit einem Getränk gefüllte Flaschen aus Glas oder aus Kunststoff oder auch Metalldosen können in Tunnelpasteuren auf an sich bekannte Weise für den Transport und die Lagerung bis hin zum Verbrauch haltbar gemacht werden. Derartige Tunnelpasteure können sowohl ein einzelnes Deck als auch zwei übereinanderliegende Decks zur Behandlung der Getränkeflaschen umfassen. Das zur Pasteurisierung dienende Prozesswasser wird dann in Sammelwannen aufgefangen, aufbereitet und erneut zur Pasteurisierung verwendet. Zum Entfernen mechanischer Verschmutzungen, wie beispielsweise von Bruchstücken zerborstener Flaschen, werden Siebe, Sedimentationswannen sowie Filter eingesetzt.

Während sich mechanische und anorganische Verunreinigungen mit herkömmlicher Technik gut aus dem Prozesswasser entfernen lassen, sind die für die Hygiene im Tunnelpasteur vor allem relevanten organische Verunreinigungen problematisch, da sie vergleichsweise aggressiv bekämpft werden müssen, beispielsweise mit desinfizierenden Chemikalien. Dies verursacht zum einen längere Produktionsunterbrechungen. Zum anderen werden störende Mikroorganismen, wie Bakterien und Pilze, durch den Einsatz der Chemikalien zwar abgetötet, verbleiben dann aber dennoch als Biomasse im Innenbereich des Tunnelpasteurs. Besonders unansehnlich und unhygienisch sind schleimige und entsprechend schwierig zu entfernende Beläge aus Biomasse, sogenannte Biofilme, auf Wänden, Böden und Installationen im Tunnelpasteur.

Zwar sind mittlerweile Aufbereitungseinheiten bekannt, mit denen das zurückgeführte Prozesswasser mittels UV-Bestrahlung desinfiziert werden kann, um die Anzahl der chemischen Reinigungszyklen zu reduzieren. Die unerwünschten Rückstände aus abgetöteter Biomasse lassen ist damit jedoch weder vermeiden noch entfernen.

Es besteht somit Bedarf für demgegenüber verbesserte Reinigungsverfahren sowie für Tunnelpasteure oder dergleichen tunnelförmige Behälterbehandlungsmaschinen mit entsprechender technischer Ausstattung.

Ein Verfahren gemäß Oberbegriff des Anspruchs 1 sowie eine Behälterbehandlungsmaschine gemäß Oberbegriff des Anspruchs 9 sind aus DE 10 2004 039 084 A1 und DE 20 2008 004 775 U1 bekannt.

KR 2015 0032037 A stellt einen Geschirrspüler vor, der mit Hydroxylradikalen desinfiziert wird.

Die gestellte Aufgabe wird mit einem Verfahren zum Reinigen einer tunnelförmigen Behälterbehandlungsmaschine, insbesondere eines Tunnelpasteurs, gemäß Anspruch 1 gelöst.

Demnach werden Hydroxylradikale in Prozesswasser der Behälterbehandlungsmaschine maschinell erzeugt und/oder eingebracht und in der Behälterbehandlungsmaschine vorhandene organische Verschmutzungen von den im Prozesswasser bereitgestellten Hydroxylradikalen zu anorganischen Verschmutzungen mineralisiert und diese aus der Behälterbehandlungsmaschine ausgewaschen.

Ferner wird das Verfahren in einem von der Behälterbehandlungsmaschine umfassten Tunnelpasteur und/oder Tunnelrückkühler und/oder Tunnelwärmer zu dessen/deren Reinigung durchgeführt.

Das Prozesswasser ist insbesondere in der Behälterbehandlungsmaschine / im Tunnelpasteur zurückgewonnenes Wasser zur Pasteurisierung der abgefüllten Getränke und/oder zur Reinigung des Tunnelpasteurs.

Die mineralisierten Verschmutzungen sind anorganisch und können vergleichsweise einfach aus der Behälterbehandlungsmaschine und insbesondere dem Tunnelpasteur ausgewaschen werden, da sie im Gegensatz zu organischen Verschmutzungen und einem Befall mit Mikroorganismen nicht zum Ausbilden schleimiger Beläge im Inneren des Tunnelpasteurs neigen. Die organischen Verbindungen werden dabei aufgebrochen und zumindest anteilig in ein mineralisches Endprodukt umgewandelt, das den Mikroorganismen keine geeignete Nahrungsgrundlage mehr bietet und ein Wachstum von Mikroorganismen daher auch unterdrückt.

Die Hydroxylradikale werden nach dem Prinzip der erweiterten Oxidation hergestellt, auch "Advanced Oxidation Process (AOP)" genannt. Bekanntermaßen sind die Endprodukte der erweiterten Oxidation von organischen Verbindungen ungiftig und umweltfreundlich.

Für die erweiterte Oxidation sind beispielsweise folgende photochemische Prozesse bekannt: Photolyse; UV-Bestrahlung von H₂O₂ und/oder O₃; Photo-Fenton-Prozess durch Kombination von UV-Strahlung, Fe³⁺ und H₂O₂.

Für die erweiterte Oxidation sind ferner folgende nicht-photochemische Prozesse bekannt: Ozonation; Ozonation in Kombination mit H₂O₂; Fenton-Prozesse durch Kombination von H₂O₂ mit Fe²⁺ oder Fe³⁺; Nassluft-Oxidation; sowie elektrochemische Oxidation.

Für die technische Umsetzung der erweiterten Oxidation sind stets zwei grundlegende Schritte nötig, nämlich zunächst die Erzeugung von Hydroxylradikalen oder anderweitigen starken Oxidationsmitteln und danach Reaktion der Hydroxylradikale mit zu entfernenden organischen Verschmutzungen.

Die Reaktion der Hydroxylradikale mit den organischen Verschmutzungen kann mehrstufig erfolgen und folgende grundsätzliche Reaktionstypen umfassen: Hydroxylradikale entreißen den jeweiligen Reaktionspartnern Wasserstoffatome, und/oder Hydroxylradikale geben an die jeweiligen Reaktionspartner Valenzelektronen ab, und/oder Hydroxylradikale werden an die jeweiligen Reaktionspartner gebunden.

Die erweiterte Oxidation führt im Idealfall zu mineralischen Endprodukten, kann aber auch als Vorstufe davon durch Zwischenprodukte bereits eine hilfreiche Unterdrückung von Mikroorganismen im Tunnelpasteur bewirken, beispielsweise durch Fragmentierung und/oder Derivatisierung organischer Moleküle. Derartige Zwischenprodukte können gegenüber den ursprünglichen organischen Verschmutzungen bereits leichter zu entfernen sein und/oder relevanten Mikroorganismen eine schlechtere Nahrungsgrundlage bieten.

Vorzugsweise umfassen die organischen Verschmutzungen Mikroorganismen und/oder Nährstoffe für die Mikroorganismen. Mit den Hydroxylradikalen können dann sowohl Mikroorganismen in der Behälterbehandlungsmaschine und insbesondere im Tunnelpasteur abgebaut werden als auch deren Wachstum durch Entzug von Nährstoffen unterdrückt werden. Anders gesagt kann sowohl die Entstehung der Mikroorganismen unterdrückt werden als auch deren Entfernung nach zumindest anteiliger Umwandlung in anorganische Verbindungen erleichtert werden.

Vorzugsweise werden die Hydroxylradikale photochemisch im Bereich einer an der Behälterbehandlungsmaschine und insbesondere am Tunnelpasteur vorhandenen Aufbereitungseinheit für Prozesswasser erzeugt. Das für den photochemischen Herstellungsprozess benötigte Licht kann dann auf einen vergleichsweise kleinen Bereich begrenzt werden, beispielsweise auf eine Rohrleitung und/oder eine Bestrahlungskammer für zurückgeführtes Prozesswasser.

Vorzugsweise werden die Hydroxylradikale hergestellt, indem ein photoreaktives bzw. UV-reaktives Oxidationsmittel dem insbesondere zurückgewonnenen Prozesswasser zugemischt und mittels UV-Licht bestrahlt wird. Das UV-Licht bewirkt dann sowohl eine Desinfektion des aufbereiteten Prozesswassers als auch die Gewinnung von Hydroxylradikalen, die sich im Prozesswasser lösen und damit im Inneren des Tunnelpasteurs verteilen lassen.

Folglich können die Hydroxylradikale ihre Reinigungswirkung im gesamten mit Prozesswasser in Berührung kommenden Innenraum des Tunnelpasteurs entfalten. Photoreaktive bzw. UV-reaktive Oxidationsmittel, wie beispielsweise H₂O₂, sind vergleichsweise einfach zu handhaben, da die Hydroxylradikale dann erst im Bestrahlungsbereich entstehen und ihre Wirkung gezielt danach entfalten können.

Vorzugsweise werden Hydroxylradikale aus H₂O₂ hergestellt. H₂O₂ ist ein zur Desinfektion von Anlagen der Trinkwasserversorgung bewährtes und auch in Abfüllanlagen für Getränke übliches Desinfektionsmittel. H₂O₂ ist vergleichsweise kostengünstig und bei Raumtemperatur und normalen Umgebungsdruck gut mit Wasser mischbar. H₂O₂ lässt sich daher einfach und präzise in unterschiedlichen Bereichen von Tunnelpasteuren dosieren und einbringen.

Vorzugsweise werden die Hydroxylradikale im Bestrahlungsbereich einer UV-Desinfektionslampe für Prozesswasser erzeugt. Zum einen lässt sich das UV-Licht sowohl für die Desinfektion des Prozesswassers als auch für die Erzeugung der Hydroxylradikale und somit doppelt nutzen. Die von den Hydroxylradikalen verursachte Schädigung und Unterdrückung von Mikroorganismen im Prozesswasser erhöht zudem auch die Effektivität der Desinfektion, da die Eindringtiefe des UV-Lichts in das Prozesswasser mit abnehmender Konzentration der Mikroorganismen ansteigt. Somit kann die Desinfektionsleistung bei gleicher elektrischer Lampenleistung erhöht werden bzw. eine bestimmte Desinfektionswirkung gegebenenfalls auch mit einer schwächeren UV-Lampe erzielt werden.

Ergänzend oder alternativ können die Hydroxylradikale nicht-photochemisch und insbesondere im Bereich einer an der Behälterbehandlungsmaschine und insbesondere am Tunnelpasteur vorhandenen Sammelwanne und/oder eines Sedimentationsbereichs für Prozesswasser erzeugt oder eingeleitet werden. Dies ist insbesondere dann vorteilhaft, wenn eine UV-Desinfektion von Prozesswasser nicht vorhanden oder möglich ist. Das Verfahren ließe sich dann gegebenenfalls auch an bereits bestehenden Tunnelpasteuren mit vergleichsweise geringem apparativem Aufwand durchführen.

Die Reinigungswirkung der Hydroxylradikale besteht darin, dass sie organische Verschmutzungen ferner molekular fragmentieren und/oder derivatisieren. Die Fragmentierung und Derivatisierung sind als Zwischenschritte der Mineralisierung organischer Verschmutzungen durch erweiterte Oxidation zu verstehen, leisten aber auch selbst einen Beitrag zum Abbau und/oder dem Auswaschen von Mikroorganismen.

Erfindungsgemäß werden die Hydroxylradikale im laufenden Arbeitsbetrieb des Tunnelpasteurs erzeugt und/oder in diesen eingebracht. Beispielsweise ist das Einleiten von H₂O₂ in Prozesswasser und die anschließende UV-Desinfektion und UV-Reaktion zu Hydroxylradikalen problemlos im laufenden Arbeitsbetrieb möglich. Somit sind Produktionsunterbrechungen für separate Reinigungszyklen zur Entfernung von Mikroorganismen und/oder darauf basierender Biomassen / Biofilme aus der Behälterbehandlungsmaschine und insbesondere dem Tunnelpasteur weitgehend entbehrlich.

Vorzugsweise werden die Hydroxylradikale in zurückgeführtem und aufbereitetem Prozesswasser gelöst und innerhalb des Behandlungstunnels des Tunnelpasteurs mittels Spritzdüsen verteilt. Das Prozesswasser mit den Hydroxylradikalen wird dann über die Spritzdüsen zur Pasteurisierung der Getränkeflaschen und/oder über zusätzliche Spritzdüsen zur Reinigung des Tunnelpasteurs in diesem verteilt. Dadurch lassen sich alle Behandlungsstufen des Tunnelpasteurs effizient mit den Hydroxylradikalen reinigen.

Die gestellte Aufgabe wird ebenso mit einer tunnelförmigen Behälterbehandlungsmaschine und insbesondere einem Tunnelpasteur gemäß Anspruch 9 gelöst. Die Behälterbehandlungsmaschine und insbesondere der Tunnelpasteur eignet sich für Getränkeflaschen oder dergleichen Behälter, also beispielsweise auch für Getränkedosen, und umfasst wenigstens eine Aufbereitungseinheit für Prozesswasser und Spritzdüsen zum Ausbringen des Prozesswassers in der Behälterbehandlungsmaschine und insbesondere im Tunnelpasteur.

Die Behälterbehandlungsmaschine umfasst einen Tunnelpasteur und/oder Tunnelrückkühler und/oder Tunnelwärmer oder besteht aus wenigstens einer dieser Einheiten.

Die Behälterbehandlungsmaschine ist beispielsweise Bestandteil einer Abfüllanlage für Getränke oder anderweitige Produkte mit vergleichbaren Hygieneanforderungen der Produktion.

Erfindungsgemäß ist die Aufbereitungseinheit zum Erzeugen und/oder zum Einbringen von Hydroxylradikalen in dem/das Prozesswasser ausgebildet, sodass in der Behälterbehandlungsmaschine vorhandene organische Verschmutzungen durch die im Prozesswasser bereitgestellten Hydroxylradikale zu anorganischen Verschmutzungen mineralisiert werden, um diese aus der Behälterbehandlungsmaschine auszuwaschen. Die Hydroxylradikale können so insbesondere im Behandlungstunnel des Tunnelpasteurs effektiv ausgebracht werden und an den benetzten Oberflächen organische Verschmutzungen abbauen und zumindest anteilig mineralisieren. Die Aufbereitungseinheit ist daher auch als Mineralisierungseinheit zu verstehen. Das Prozesswasser kann zur Pasteurisierung der Getränkeflaschen und/oder zur Reinigung des Tunnelpasteurs dienen.

Somit lassen sich unansehnliche und unhygienische Biofilme mit lebenden und abgetöteten Mikroorganismen effizient von den Innenwänden des Behandlungstunnels, von Oberflächen der darin vorhandenen Installationen sowie aus Leitungen für das Prozesswasser entfernen.

Die Behälterbehandlungsmaschine und insbesondere der Tunnelpasteur ist demnach zur Durchführung des Verfahrens nach wenigstens einer der voranstehend beschriebenen Ausführungsformen ausgebildet.

Vorzugsweise ist die Aufbereitungseinheit (Mineralisierungseinheit) zur nicht-photochemischen Erzeugung und/oder zur Einbringung von Hydroxylradikalen an einer in der Behälterbehandlungsmaschine und insbesondere im Tunnelpasteur ausgebildeten Sammelwanne für Prozesswasser und/oder zur photochemischen Erzeugung von Hydroxylradikalen in einem an der Behälterbehandlungsmaschine und insbesondere am Tunnelpasteur vorhandenen Aufbereitungskreislauf für Prozesswasser ausgebildet. Auf diese Weise lassen sich nicht-photochemische und photochemische Erzeugung von Hydroxylradikalen besonders effizient durchführen oder auch miteinander kombinieren.

Vorzugsweise umfasst die Behälterbehandlungsmaschine und insbesondere der Tunnelpasteur bzw. die Aufbereitungseinheit wenigstens eine UV-Lampe zur Bestrahlung von Prozesswasser und eine insbesondere in einem Zulaufbereich zur UV-Lampe ausgebildete Dosiereinrichtung für ein photoreaktives bzw. UV-reaktives Oxidationsmittel, insbesondere H₂O₂, zur photochemischen Erzeugung der Hydroxylradikale. UV-Bestrahlung eignet sich sowohl zur photochemischen Erzeugung der Hydroxylradikale als auch zur Desinfektion des Prozesswassers. Im Zulaufbereich zur UV-Lampe lässt sich das Oxidationsmittel gezielt dosieren und gleichmäßig im Prozesswasser verteilen, bevor es unmittelbar anschließend mittels UV-Licht in Hydroxylradikale umgewandelt wird.

Vorzugsweise ist die Dosiereinrichtung innerhalb einer Fließstrecke von höchstens 1 m vor der UV-Lampe angeordnet. Damit lässt sich vermeiden, dass das photoreaktive bzw. UV-reaktive Oxidationsmittel, wie beispielsweise H₂O₂, das auch ohne UV-Bestrahlung bereits oxidierend wirkt, bereits auf dem Weg zur UV-Lampe mit organischen Verschmutzungen, wie beispielsweise einem Biofilm, reagiert. Derartige Vorabreaktionen mit organischen Verschmutzungen und insbesondere mit Mikroorganismen sind eher unerwünscht, da sie keine Mineralisierung der organischen Verschmutzungen mit den voranstehend genannten Vorteilen bewirken und stattdessen die Effizienz der Mineralisierung reduzieren können.

Folglich eignen sich vor allem Rohrleitungen mit einer Länge von höchstens 1 m zwischen der Einleitung des photoreaktiven Oxidationsmittels und dem Eingang zur von der UV-Lampe ausgeleuchteten Desinfektionskammer. Besonders vorteilhaft ist ein derartiger Fließabstand von höchstens 0,5 m.

Vorzugsweise ist die UV-Lampe Bestandteil einer Aufbereitungseinheit zur UV-Desinfektion und zur Filterung von Prozesswasser. Die UV-Lampe lässt sich dann sowohl zur Desinfektion als auch zur Erzeugung von Hydroxylradikalen verwenden. Durch die Erzeugung der Hydroxylradikale lässt sich wiederum die Effektivität der UV-Lampe durch verbesserte Eindringtiefe des UV-Lichts in das zu desinfizierende Prozesswasser verbessern.

Vorzugsweise umfasst die Aufbereitungseinheit (Mineralisierungseinheit) eine im Bereich einer Sammelwanne, insbesondere eines Sedimentationsbereichs für das Prozesswasser angeordnete Dosiereinrichtung zur Beimengung einer Mineralisierungslösung mit Hydroxylradikalen zum Prozesswasser. Dort kann sich das Oxidationsmittel gleichmäßig im aufgefangenen Prozesswasser verteilen und gemeinsam mit diesem zur Aufbereitungseinheit zurückgeführt werden. Die Dosiereinrichtung könnte auch in einem Ablaufbereich aus der Sammelwanne bzw. des Sedimentationsbereichs zur Aufbereitungseinheit hin angeordnet werden. Die Mineralisierungslösung basiert dann vorzugsweise auf einem Oxidationsmittel zur nicht-photochemischen Bereitstellung von Hydroxylradikalen.

Bevorzugte Ausführungsformen der Erfindung sind zeichnerisch dargestellt. Es zeigen:
- Figur 1: einen schematischen Querschnitt durch einen Tunnelpasteur; und
- Figur 2: ein Reaktionsschema zur erweiterten photochemischen Oxidation organischer Verunreinigungen.

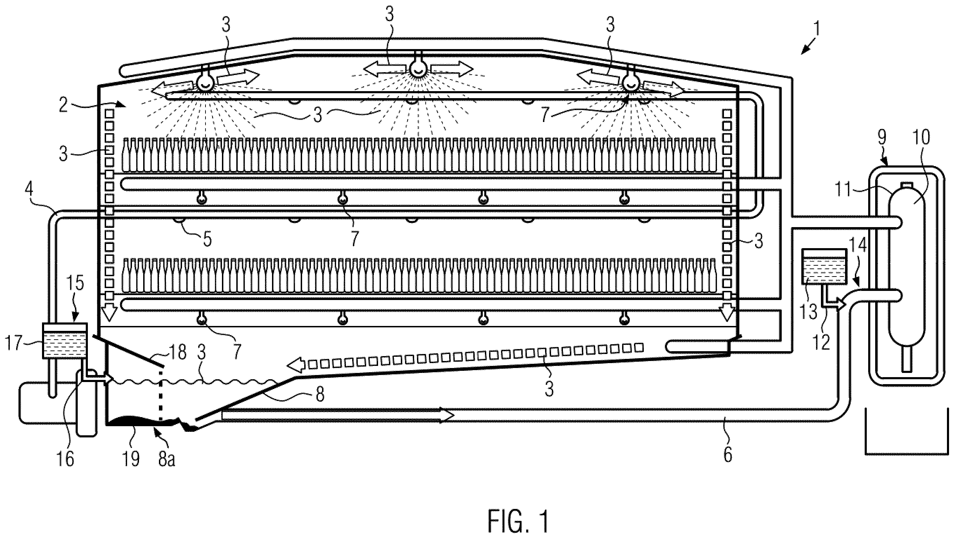

Wie die Figur 1 im schematischen Querschnitt einer beispielhaft als Tunnelpasteur ausgebildeten Behälterbehandlungsmaschine 1 erkennen lässt, umfasst diese einen Behandlungstunnel 2 zur Berieselung von Getränkeflaschen oder anderer mit einem Produkt gefüllter Behälter mit Prozesswasser 3, das je nach Behandlungszone heiß oder kalt temperiert ist. Dies ist ebenso bekannt wie der beispielhaft dargestellte Transport der Getränkeflaschen auf zwei übereinanderliegenden Behandlungsdecks.

Die Behälterbehandlungsmaschine 1 ist tunnelförmig und kann alternativ oder ergänzend einen Tunnelrückkühler und/oder einen Tunnelwärmer umfassen bzw. aus wenigstens einer dieser Einheiten bestehen.

Die Behälterbehandlungsmaschine 1 und insbesondere der Tunnelpasteur umfasst ferner einen Behandlungskreislauf 4 mit Spritzdüsen 5 für die Behandlung der Getränkeflaschen 2 mit dem Prozesswasser 3 sowie einen Aufbereitungskreislauf 6 mit Spritzdüsen 7 zur Reinigung des Behandlungstunnels 1a mit aufbereitetem Prozesswasser 3.

Das von den Düsen 5, 7 versprühte Prozesswasser 3 wird in einer Sammelwanne 8 aufgefangen und kann von dort sowohl in Richtung des Behandlungskreislaufs 4, beispielsweise zwecks Erhitzung oder Kühlung, als auch in Richtung des Aufbereitungskreislaufs 6 abgezogen werden.

Der Aufbereitungskreislauf 6 umfasst eine erste Aufbereitungseinheit 9 zur UV-Desinfektion des Prozesswassers 3 und zur photochemischen Erzeugung von Hydroxylradikalen im Prozesswasser 3 für eine anschließende Mineralisierung organischer Verschmutzungen im Behandlungstunnel 2.

Die erste Aufbereitungseinheit 9 umfasst eine UV-Lampe 10 in einer Desinfektionskammer 11, welche von aufzubereitendem Prozesswasser 3 durchströmt wird, und eine erste Dosiereinrichtung 12 für ein photoreaktives bzw. UV-reaktives Oxidationsmittel 13, insbesondere H₂O₂. Die erste Dosiereinrichtung 12 ist vorzugsweise in einem Zulaufbereich 14 zur Desinfektionskammer 11 bzw. zur UV-Lampe 10 angeordnet, insbesondere innerhalb einer Fließstrecke von höchstem 1 m bis zur Desinfektionskammer 11.

Das aufzubereitende Prozesswasser 3 wird in der Desinfektionskammer 11 von der UV-Lampe 10 bestrahlt und dadurch desinfiziert. Zusätzlich bewirkt die UV-Lampe 10 eine Reaktion des von der ersten Dosiereinrichtung 12 in das Prozesswasser 3 dosierten photoreaktiven Oxidationsmittels 13 zu Hydroxylradikalen. Vorzugsweise wird das Prozesswasser 3 in der ersten Aufbereitungseinheit 9 zudem auf herkömmliche Weise gefiltert.

Die derart photochemisch erzeugten Hydroxylradikale lösen sich im Prozesswasser 3 und werden in diesem über den Aufbereitungskreislauf 6 zu den Spritzdüsen 7 gepumpt. Die Spritzdüsen 7 sind auf geeignete Weise im Behandlungstunnel 2 verteilt, um zu reinigende Oberflächen an Innenwänden, Installationen oder dergleichen mit dem aufbereiteten Prozesswasser 3 zu besprühen.

Dadurch kommen die Hydroxylradikale mit allen Oberflächen der Behälterbehandlungsmaschine 1 und insbesondere des Tunnelpasteurs in Kontakt, die auch vom Prozesswasser 3 benetzt werden. Dort vorhandene organische Verschmutzungen einschließlich Mikroorganismen reagieren mit den so eingebrachten Hydroxylradikalen und werden durch erweiterte Oxidation sukzessive abgebaut und zumindest anteilig mineralisiert. Somit wird beispielsweise der Bildung schleimartiger und/oder fest auf den Oberflächen der Behälterbehandlungsmaschine 1 und insbesondere des Tunnelpasteurs haftender Biofilme entgegengewirkt.

Durch schrittweise Fragmentierung, Derivatisierung und Mineralisierung organischer Verbindungen werden Mikroorganismen nicht nur abgetötet sondern auch zu den einfacher zu entfernenden Zwischenprodukten und Endprodukten der erweiterten Oxidation abgebaut. Ferner wird Bakterien, Pilzen oder dergleichen Mikroorganismen Nahrung entzogen. Folglich ist eine umfassende Reduzierung und Entfernung unansehnlicher und unhygienischer Biofilme im Inneren der Behälterbehandlungsmaschine 1 und insbesondere des Tunnelpasteurs möglich.

Die erste Aufbereitungseinheit 9 dient somit einer maschinellen und insbesondere möglichst vollständigen Mineralisierung organischer Verschmutzungen durch erweiterte Oxidation in der Behälterbehandlungsmaschine 1 und insbesondere im Tunnelpasteur.

Derartige organische Verschmutzungen können prinzipiell im gesamten Behandlungstunnel 2 aufgrund von Flaschenbruch oder einem anderweitigen Austreten abgefüllter Getränke auftreten. Beispielsweise können flüchtige Bestandteile der Getränke in der Behälterbehandlungsmaschine 1 und insbesondere im Tunnelpasteur nicht zuletzt wegen der vergleichsweise hohen Behandlungstemperaturen verdampfen und sich an den Innenwänden und/oder Installationen des Behandlungstunnels 2 niederschlagen. Im Behandlungstunnel 2 herrschen zudem je nach Behandlungszone gute klimatische Brutbedingungen für Mikroorganismen. Somit können sich Biofilme prinzipiell in vielen Bereichen des Behandlungstunnels 2 bilden. Betroffen von den organischen Verschmutzungen sind beispielsweise die Sammelwanne 8 sowie alle Rohrleitungen und Installationen, durch die mit Mikroorganismen kontaminiertes Prozesswasser 3 fließt.

Die Figur 1 zeigt schematisch die im unteren Bereich des Behandlungstunnels 2 ausgebildete Sammelwanne 8 für Prozesswasser 3 mit einem Sedimentationsbereich 8a, in dem sich kleinere Fremdkörper, Schwebstoffe oder anderweitig unlösliche Verschmutzungen absetzen können, sowie eine zweite Aufbereitungseinheit 15 mit einer zweiten Dosiereinrichtung 16 zur Beimengung einer Hydroxylradikale enthaltenden Mineralisierungslösung 17 zum Abbau organischer Verschmutzungen.

Die Hydroxylradikale in der Mineralisierungslösung 17 werden vorzugsweise mittels nicht-photochemischer Reaktion aus einem geeigneten Oxidationsmittel gewonnen. Denkbar sind aber auch photoreaktive Oxidationsmittel und/oder eine photochemische Erzeugung der Mineralisierungslösung 17.

Die zweite Aufbereitungseinheit 15 ist vorzugsweise am Sedimentationsbereich 8a angeordnet zugunsten einer gleichmäßigen Durchmischung des Prozesswassers 3 und der dosierten Mineralisierungslösung 17. Über dem Sedimentationsbereich 8a ist beispielsweise ein herkömmliches Sieb 18 zum Zurückhalten von Glassplittern oder anderweitigen größeren Fremdkörpern aus dem zu sammelnden Prozesswasser 3 angeordnet.

Auch die zweite Aufbereitungseinheit 15 dient einer maschinellen und insbesondere möglichst vollständigen Mineralisierung organischer Verschmutzungen durch erweiterte Oxidation in der Behälterbehandlungsmaschine 1 und insbesondere im Tunnelpasteur.

Im gezeigten Beispiel ist die erste Aufbereitungseinheit 9 zur photochemischen Erzeugung von Hydroxylradikalen und deren Beimengung zu aufzubereitendem Prozesswasser 3 ausgebildet, die zweite Aufbereitungseinheit 15 dagegen zur nicht-photochemischen Erzeugung und/oder Beimengung von Hydroxylradikalen zum Prozesswasser 3. Eine der beiden Aufbereitungseinheiten 9, 15 kann zur Durchführung der Mineralisierung organischer Verschmutzungen bereits ausreichend sein.

Denn in beiden Fällen bewirken die so bereitgestellten Hydroxylradikale eine Umwandlung organischer Verschmutzungen, wie beispielsweise Mikroorganismen und/oder Nährstoffen für die Mikroorganismen, zumindest teilweise in mineralische Endprodukte. Diese mineralischen Endprodukte können sich beispielsweise im Sedimentationsbereich 8a des Sammelwanne 8 absetzen und sind in der Figur 1 schematisch und beispielhaft als mineralische Ablagerung 19 dargestellt. Solche mineralischen Ablagerungen 19 lassen sich vergleichsweise einfach aus der Behälterbehandlungsmaschine 1 und insbesondere dem Tunnelpasteur mit seinen Installationen und Rohrleitungen entfernen, im Gegensatz zu Biofilmen mit lebenden und/oder abgetöteten Mikroorganismen, die aufgrund ihrer schleimartigen Konsistenz nur mit großen Aufwand und/oder nur unvollständig entfernt werden können.

Beide Aufbereitungseinheiten 9, 15 können im Sinne der vorliegenden Erfindung somit auch als Mineralisierungseinheiten verstanden werden, um Mikroorganismen und daraus resultierende abgestorbene Biomasse abzubauen und möglichst vollständig in vergleichsweise einfach zu entfernende Zwischenprodukte und mineralische Endprodukte mit geringem Nährwert für Mikroorganismen umzuwandeln.

Im Vergleich zu herkömmlichen chemischen Reinigungsverfahren und/oder dem Auskochen von Behälterbehandlungsmaschinen 1 und insbesondere Tunnelpasteuren kann somit die Menge unansehnlicher und hygienisch bedenklicher Biofilme nachhaltig reduziert werden. Personalaufwand und Produktionsunterbrechungen für Reinigungszyklen lassen sich so erheblich reduzieren.

Mit den Aufbereitungseinheiten (Mineralisierungseinheiten) 9, 15 und den Spritzdüsen 5, 7 lassen sich zudem die hygienisch relevanten Bereiche der Behälterbehandlungsmaschine 1 und insbesondere des Tunnelpasteurs über das Prozesswasser 3 weitgehend unabhängig vom Ort der Erzeugung und/oder Einbringung der Hydroxylradikale reinigen.

Die photochemische Erzeugung von Hydroxylradikalen wäre prinzipiell auch unabhängig von einer UV-Desinfektion/Filterung möglich, nämlich mit wenigstens einer separaten UV-Lampe (nicht dargestellt) im Aufbereitungskreislauf 6 und/oder im Behandlungstunnel 2. Auch dort könnten im Prozesswasser 3 gelöste photoreaktive Oxidationsmittel, wie beispielsweise H₂O₂, prinzipiell mittels UV-Lampen (nicht dargestellt) bestrahlt werden und dadurch gegebenenfalls auch direkt an der zu reinigenden Ort und Stelle zu mineralisierenden Hydroxylradikalen reagieren. Das einer photochemischen Reaktion zu Hydroxylradikalen zugrundeliegende photoreaktive Oxidationsmittel könnte dann mit dem Prozesswasser 3 beispielsweise über den Behandlungskreislauf 4 und die Spritzdüsen 5 ausgebracht und an geeigneter Stelle gezielt mit UV-Licht bestrahlt werden.

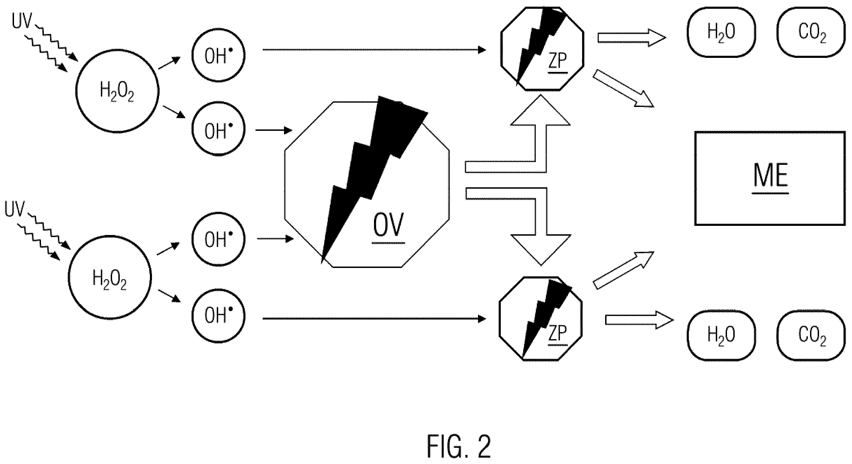

Die Figur 2 zeigt ein prinzipielles Reaktionsschema der erweiterten Oxidation mittels der Hydroxylradikale OH·. Beispielhaft ist die erweiterte Oxidation ausgehend von H₂O₂ und UV-Licht UV dargestellt. Demnach spaltet das UV-Licht UV zuerst H₂O₂-Moleküle in jeweils zwei Hydroxylradikale OH· auf.

Diese reagieren dann mit einer organischen Verschmutzung OV zu organischen Zwischenprodukten ZP, im Beispiel durch Fragmentierung der organischen Verschmutzung OV. Bei den Zwischenprodukten ZP kann es sich um unterschiedliche organische Moleküle handeln.

Die organischen Zwischenprodukte ZP können dann in weiteren Reaktionen mit Hydroxylradikalen OH· zu einem oder mehreren mineralischen Endprodukten ME reagieren, beispielsweise unter Abspaltung von H₂O und CO₂.

Hierbei soll die Figur 2 lediglich schematisch vermitteln, welches Reaktionsschema der Mineralisierung der organischen Verschmutzungen OV zugrunde liegt. Die vorteilhafte Reinigungswirkung ergibt sich aus einer Kombination von Zwischenschritten und der abschließenden Reaktion zu einem mineralischen Endprodukt ME. Nicht alle organische Verunreinigungen müssen demnach vollständig in ein mineralisches Endprodukt ME umgewandelt werden. Auch die organischen Zwischenprodukte ZP, die beispielsweise durch schematisch dargestellte Fragmentierung und/oder durch Derivatisierung entstehen können, sind leichter zu entfernen als ursprünglich vorhandene organische Verschmutzungen OV und entziehen Mikroorganismen oder dergleichen Biomasse in der Behälterbehandlungsmaschine 1 und insbesondere im Tunnelpasteur zudem Nahrung.

Hierbei ist die beispielhaft dargestellte photochemische Erzeugung der Hydroxylradikale OH· wegen der guten Handhabbarkeit von H₂O₂ im Bereich von Abfüllanlagen oder dergleichen Anlagen im Lebensmittelbereich und dem gut kontrollierbaren Einsatz von UV-Licht UV vorteilhaft. Insbesondere die Kombination mit der Desinfektionswirkung des UV-Lichts UV auf das aufzubereitende Prozesswasser 3 ist für den Betrieb der Behälterbehandlungsmaschine 1 und insbesondere des Tunnelpasteurs besonders effizient. Zum einen kann die UV-Lampe 10 gleichzeitig sowohl zur Desinfektion des Prozesswassers 3 als auch zur Erzeugung der Hydroxylradikale OH· verwendet werden. Zum anderen verbessert sich durch den Abbau organischer Verschmutzungen OV die Effizienz der UV-Lampe 10 für die Desinfektion des Prozesswassers 3.

Beide Prozesse, nämlich die UV-Desinfektion und die photochemische Erzeugung der Hydroxylradikale OH·, können während des laufenden Betriebs der Behälterbehandlungsmaschine 1 und insbesondere des Tunnelpasteurs durchgeführt werden, wobei durch die Verteilung des Prozesswassers 3 in der Behälterbehandlungsmaschine 1 und insbesondere im Tunnelpasteur auch eine umfassende Mineralisierung und damit Reduzierung von Biomasse in der Behälterbehandlungsmaschine 1 und insbesondere im Tunnelpasteur gegeben ist.

Hierfür eignen sich sowohl der Behandlungskreislauf 4 mit seinen Spritzdüsen 5 als auch der separate Aufbereitungskreislauf 6 mit seinen Spritzdüsen 7. Ebenso können wahlweise Aufbereitungseinheiten (Mineralisierungseinheiten) 9, 15 mit photochemischer und/oder nichtphotochemischer Erzeugung und/oder Einbringung von Hydroxylradikalen zum Einsatz kommen.

Beispielsweise kann die Mineralisierung organischer Verschmutzungen OV im laufenden Betrieb wie folgt durchgeführt werden.

Die zu pasteurisierenden Getränkeflaschen werden laufend dem Behandlungstunnel 2 zugeführt und auf wenigstens einem Behandlungsdeck kontinuierlich durch Behandlungszonen zur Erhitzung oder Kühlung der Getränkeflaschen gefördert. Hierbei werden sie mit dem geeignet temperierten Prozesswasser 3 berieselt. Das Prozesswasser 3 läuft durch die Decks und wird von der Sammelwanne 8 aufgefangen und ihrem Sedimentationsbereich 8a gesammelt. Von dort wird das mechanisch geklärte Prozesswasser 3 teilweise in den Aufbereitungskreislauf 6 und teilweise in den Behandlungskreislauf 4 abgezogen.

Im Aufbereitungskreislauf 6 wird Prozesswasser 3 in geeigneten zeitlichen Abständen oder kontinuierlich zur ersten Aufbereitungseinheit 9 gepumpt und von der UV-Lampe 10 bestrahlt. Im Zulaufbereich 14 zur UV-Lampe 10 wird dem Prozesswasser 3 das photoreaktive Oxidationsmittel 13, insbesondere H₂O₂, gezielt beigemengt und mit dem Prozesswasser 3 in den Bestrahlungsbereich der UV-Lampe 10 transportiert. Dort wird das Prozesswasser 3 mittels UV-Licht UV desinfiziert. Dabei werden Hydroxylradikale OH· aus dem photoreaktiven Oxidationsmittel 13 gebildet. Das derart aufbereitete und vorzugsweise ferner mechanisch gefilterte Prozesswasser 3 wird dann weiter zu den Spritzdüsen 7 gepumpt und von diesen im Behandlungstunnel 2 verteilt.

Hierbei kann die UV-Desinfektion im Prinzip beliebig durch die photochemische Erzeugung von Hydroxylradikalen OH· im Prozesswasser 3 ergänzt werden. Beispielsweise könnten identische oder fest gekoppelte Aufbereitungs- und Reinigungszyklen für beide Aufbereitungsprozesse vorgegeben werden. Ebenso könnte eine Beimengung des photoreaktiven Oxidationsmittels 13 je nach tatsächlichem Bedarf oder Reinigungsplan der UV-Desinfektion gezielt zugeschaltet werden.

Im Bestrahlungstunnel 2 vorhandene organische Verschmutzungen OV werden den Hydroxylradikalen OH· schließlich durch Benetzen mit dem derart aufbereiteten Prozesswasser 3 ausgesetzt und folglich mittels Fragmentierung und/oder Derivatisierung zu organischen Zwischenprodukten ZP sowie zumindest teilweise zu wenigstens einem mineralisches Endprodukt ME abgebaut.

Die Zwischenprodukte ZP und die mineralischen Endprodukte ME der erweiterten Oxidation sind im Prozesswasser 3 prinzipiell löslich und/oder können von diesem mechanisch mitgerissen werden und könnten sich dann im Bereich der Sammelwanne 8 absetzen. Dies ermöglicht eine kontinuierliche oder bedarfsorientierte Reinigung der Behälterbehandlungsmaschine 1 und insbesondere des Tunnelpasteurs 1 insgesamt während des laufenden Produktionsbetriebs. Bewuchs mit Mikroorganismen oder darauf basierende Biofilme werden entfernt und/oder unterdrückt.

Ergänzend oder alternativ kann eine insbesondere nicht-photochemisch erzeugte Mineralisierungslösung 17 mit Hydroxylradikalen OH· vorzugsweise im Bereich der Sammelwanne 8 und insbesondere ihres Sedimentationsbereichs 8a dem Prozesswasser 3 dosiert beigemengt und so prinzipiell beliebig in Rohrleitungen und/oder dem Behandlungstunnel 2 verteilt werden.

Somit lässt sich die Mineralisierung organischer Verschmutzungen OV insbesondere unter Reduzierung oder gar Vermeidung von Bewuchs mit Mikroorganismen effizient mit wenigstens einer der Aufbereitungseinheiten (Mineralisierungseinheiten) 9, 15 durchführen. Diese könnten je nach apparativer Voraussetzung an bestehenden Tunnelpasteuren, Tunnelrückkühlern und/oder Tunnelwärmern auch nachgerüstet werden.

## Patentansprüche

1. Verfahren zum Reinigen einer tunnelförmigen Behälterbehandlungsmaschine (1), durchgeführt in einem von der Behälterbehandlungsmaschine umfassten Tunnelpasteur und/oder Tunnelrückkühler und/oder Tunnelwärmer, **dadurch gekennzeichnet, dass** Hydroxylradikale (OH') in Prozesswasser (3) der Behälterbehandlungsmaschine (1) im laufenden Arbeitsbetrieb der Behälterbehandlungsmaschine (1) erzeugt und/oder eingebracht werden, und wobei in der Behälterbehandlungsmaschine (1) vorhandene organische Verschmutzungen (OV) von den im Prozesswasser (3) bereitgestellten Hydroxylradikalen (OH') zu anorganischen Verschmutzungen mineralisiert werden, und wobei diese aus der Behälterbehandlungsmaschine (1) ausgewaschen werden.

2. Verfahren nach Anspruch 1, wobei die organischen Verschmutzungen (OV) Mikroorganismen und/oder Nährstoffe für die Mikroorganismen umfassen.

3. Verfahren nach Anspruch 1 oder 2, wobei die Hydroxylradikale (OH') photochemisch im Bereich einer an der Behälterbehandlungsmaschine (1), insbesondere am Tunnelpasteur, vorhandenen Aufbereitungseinheit (9) für Prozesswasser (3) erzeugt werden.

4. Verfahren nach einem der vorigen Ansprüche, wobei die Hydroxylradikale (OH') hergestellt werden, indem ein photoreaktives Oxidationsmittel (13) insbesondere zurückgewonnenem Prozesswasser (3) zugemischt und mittels UV-Licht (UV) bestrahlt wird.

5. Verfahren nach einem der vorigen Ansprüche, wobei die Hydroxylradikale (OH·) aus H₂O₂ hergestellt werden.

6. Verfahren nach wenigstens einem der vorigen Ansprüche, wobei die Hydroxylradikale (OH·) im Bestrahlungsbereich einer UV-Lampe (10) für die Desinfektion von Prozesswasser (3) erzeugt werden.

7. Verfahren nach wenigstens einem der vorigen Ansprüche, wobei die Hydroxylradikale (OH') nicht-photochemisch im Bereich einer an der Behälterbehandlungsmaschine (1), insbesondere am Tunnelpasteur, vorhandenen Sammelwanne (8) für Prozesswasser (3) erzeugt und/oder eingeleitet werden.

8. Verfahren nach wenigstens einem der vorigen Ansprüche, wobei die Hydroxylradikale (OH') in zurückgeführtem und aufbereitetem Prozesswasser (3) innerhalb des Behandlungstunnels (2) der Behälterbehandlungsmaschine (1), insbesondere des Tunnelpasteurs, mittels Spritzdüsen (5, 7) verteilt werden.

9. Behälterbehandlungsmaschine (1) für Getränkeflaschen oder dergleichen gefüllte Behälter, mit einer Aufbereitungseinheit (9, 15) für Prozesswasser (3) und mit Spritzdüsen (5, 7) zum Ausbringen des Prozesswassers in der tunnelförmig ausgebildeten Behälterbehandlungsmaschine (1), welche einen Tunnelpasteur und/oder Tunnelrückkühler und/oder einen Tunnelwärmer umfasst, **dadurch gekennzeichnet, dass** die Aufbereitungseinheit (9, 15) zum Erzeugen und/oder Einbringen von Hydroxylradikalen (OH') in das Prozesswasser (3) im laufenden Arbeitsbetrieb der Behälterbehandlungsmaschine (1) derart ausgebildet ist, dass in dieser vorhandene organische Verschmutzungen (OV) durch die im Prozesswasser (3) bereitgestellten Hydroxylradikale (OH') zu anorganischen Verschmutzungen mineralisiert werden, um diese aus der Behälterbehandlungsmaschine (1) auszuwaschen.

10. Behälterbehandlungsmaschine nach Anspruch 9, wobei die Aufbereitungseinheit (9, 15) zur nicht-photochemischen Erzeugung und/oder zur Einbringung von Hydroxylradikalen (OH') an einer in der Behälterbehandlungsmaschine (1), insbesondere im Tunnelpasteur, ausgebildeten Sammelwanne (8) und/oder zur photochemischen Erzeugung von Hydroxylradikalen (OH') in einem an der Behälterbehandlungsmaschine (1), insbesondere am Tunnelpasteur, vorhandenen Aufbereitungskreislauf (6) für Prozesswasser (3) ausgebildet ist.

11. Behälterbehandlungsmaschine nach Anspruch 9 oder 10, ferner mit wenigstens einer UV-Lampe (10) zur Bestrahlung von Prozesswasser (3) und mit einer in einem Zulaufbereich (14) zur UV-Lampe (10) ausgebildeten Dosiereinrichtung (12) für ein photoreaktives Oxidationsmittel (13).

12. Behälterbehandlungsmaschine nach Anspruch 11, wobei die Dosiereinrichtung (12) in einem Leitungsabstand von höchstens 1 m vor der UV-Lampe (10) angeordnet ist.

13. Behälterbehandlungsmaschine nach Anspruch 11 oder 12, wobei die UV-Lampe (10) Bestandteil einer Aufbereitungseinheit (9) zur UV-Desinfektion von Prozesswasser (3) ist.

14. Behälterbehandlungsmaschine nach einem der Ansprüche 9 bis 13, wobei die Aufbereitungseinheit (15) eine im Bereich einer Sammelwanne (8) für das Prozesswasser (3) angeordnete Dosiereinrichtung (16) zur Beimengung einer Mineralisierungslösung (17) mit Hydroxylradikalen (OH*) zum Prozesswasser (3) umfasst.

## Claims

1. A method for cleaning a tunnel-type container treatment machine (1), carried out in a tunnel pasteurizer and/or tunnel recooler and/or tunnel heater comprised by the container treatment machine, **characterized in that** hydroxyl radicals (OH·) are generated and/or introduced into the process water (3) of the container treatment machine (1) are generated and/or introduced during ongoing operation of the container treatment machine (1), and wherein organic contaminants (OV) present in the container treatment machine (1) are mineralized into inorganic contaminants by the hydroxyl radicals (OH·) provided in the process water (3), and wherein these are washed out of the container treatment machine (1).

2. The method according to claim 1, wherein the organic contaminants (OV) comprise microorganisms and/or nutrients for the microorganisms.

3. The method according to claim 1 or 2, wherein the hydroxyl radicals (OH·) are generated photochemically in the region of a conditioning unit (9) for process water (3) present on the container treatment machine (1), in particular on the tunnel pasteurizer.

4. The method according to any of the preceding claims, wherein the hydroxyl radicals (OH·) are generated by admixing a photoreactive oxidizing agent (13) with, in particular, recovered process water (3) and irradiating the mixture with UV light (UV).

5. The method according to one of the preceding claims, wherein the hydroxyl radicals (OH·) are generated from H₂O₂.

6. The method according to at least one of the preceding claims, wherein the hydroxyl radicals (OH·) are generated in the irradiation zone of a UV lamp (10) for the disinfection of process water (3).

7. The method according to at least one of the preceding claims, wherein the hydroxyl radicals (OH·) are generated and/or introduced non-photochemically in the region of a collection trough (8) for process water (3) provided on the container treatment machine (1), in particular on the tunnel pasteurizer.

8. The method according to at least one of the preceding claims, wherein the hydroxyl radicals (OH·) are distributed by means of spray nozzles (5, 7) in recirculated and treated process water (3) within the treatment tunnel (2) of the container treatment machine (1), in particular the tunnel pasteurizer.

9. Container treatment machine (1) for beverage bottles or similar filled containers, comprising a conditioning unit (9, 15) for process water (3) and spray nozzles (5, 7) for discharging the process water within the tunnel-shaped container treatment machine (1), which comprises a tunnel pasteurizer and/or tunnel recooler and/or a tunnel heater, **characterized in that** the conditioning unit (9, 15) for generating and/or introducing hydroxyl radicals (OH·) into the process water (3) during ongoing operation of the container treatment machine (1) is designed such that organic contaminants (OV) present therein are mineralized into inorganic contaminants by the hydroxyl radicals (OH·) (3) provided in the process water, in order to wash them out of the container treatment machine (1).

10. The container treatment machine according to claim 9, wherein the conditioning unit (9, 15) for the non-photochemical generation and/or introduction of hydroxyl radicals (OH·) is located at a collection trough (8) formed within the container treatment machine (1), in particular in the tunnel pasteurizer, and/or for the photochemical generation of hydroxyl radicals (OH·) in a conditioning circuit (6) for process water (3) provided on the container treatment machine (1), in particular on the tunnel pasteurizer.

11. The container treatment machine according to claim 9 or 10, further comprising at least one UV lamp (10) for irradiating process water (3) and a dosing device (12) for a photoreactive oxidizing agent (13), the dosing device (12) being formed in an inlet region (14) to the UV lamp (10).

12. The container treatment machine according to claim 11, wherein the dosing device (12) is arranged at a distance of no more than 1 m in front of the UV lamp (10).

13. The container treatment machine according to claim 11 or 12, wherein the UV lamp (10) is part of a conditioning unit (9) for UV disinfection of process water (3).

14. The container treatment machine according to any one of claims 9 through 13, wherein the conditioning unit (15) comprises a dosing device (16) arranged in the vicinity of a collection trough (8) for the process water (3) for adding a mineralization solution (17) containing hydroxyl radicals (OH·) to the process water (3).

## Revendications

1. Procédé de nettoyage d'une machine de traitement de récipients (1) en forme de tunnel, mis en œuvre au sein d'un pasteurisateur tunnel et/ou d'un refroidisseur tunnel et/ou d'un réchauffeur tunnel contenu dans la machine de traitement de récipients, **caractérisé en ce que** des radicaux hydroxyle (OH') sont produits et/ou introduits dans l'eau de procédé (3) de la machine de traitement de récipients (1) pendant le fonctionnement de la machine de traitement de récipients (1), et dans lequel des impuretés organiques (OV) présentes dans la machine de traitement de récipients (1) sont minéralisées par les radicaux hydroxyle (OH') fournis dans l'eau de procédé pour donner des impuretés inorganiques (3), et dans lequel lesdites impuretés inorganiques sont éliminées de la machine de traitement de récipients (1) par lavage.

2. Procédé selon la revendication 1, dans lequel les impuretés organiques (OV) comprennent des micro-organismes et/ou des nutriments pour les micro-organismes.

3. Procédé selon la revendication 1 ou 2, dans lequel les radicaux hydroxyle (OH') sont produits de manière photochimique dans la région d'une unité de préparation (9) présente au niveau de la machine de traitement de récipients (1), en particulier au niveau du pasteurisateur tunnel, et destinée à l'eau de procédé (3).

4. Procédé selon l'une quelconque des revendications précédentes, dans lequel les radicaux hydroxyle (OH') sont produits en mélangeant un agent oxydant photoréactif (13) avec de l'eau de procédé (3), en particulier recyclée, et en l'irradiant au moyen de lumière UV (UV).

5. Procédé selon l'une quelconque des revendications précédentes, dans lequel les radicaux hydroxyle (OH') sont produits à partir de H₂O₂.

6. Procédé selon au moins l'une quelconque des revendications précédentes, dans lequel les radicaux hydroxyle (OH') sont générés dans la région d'irradiation d'une lampe UV (10) destinée à la désinfection de l'eau de procédé (3).

7. Procédé selon au moins l'une quelconque des revendications précédentes, dans lequel les radicaux hydroxyle (OH') sont produits de manière non photochimique et/ou introduits dans la région d'une cuve de collecte (8) présente au niveau de la machine de traitement de récipients (1), en particulier au niveau du pasteurisateur tunnel, et destinée à l'eau de procédé (3).

8. Procédé selon au moins l'une quelconque des revendications précédentes, dans lequel les radicaux hydroxyle (OH') sont répartis dans de l'eau de procédé (3) recyclée et préparée, à l'intérieur du tunnel de traitement (2) de la machine de traitement de récipients (1), en particulier du pasteurisateur tunnel, au moyen de buses de pulvérisation (5, 7).

9. Machine de traitement de récipients (1) pour des bouteilles de boissons ou des récipients remplis similaires, comprenant une unité de préparation (9, 15) destinée à de l'eau de procédé (3) et des buses de pulvérisation (5, 7) permettant d'épandre l'eau de procédé dans la machine de traitement de récipients (1) réalisée en forme de tunnel, qui comprend un pasteurisateur tunnel et/ou un refroidisseur tunnel et/ou un réchauffeur tunnel, **caractérisée en ce que** l'unité de préparation (9, 15) permettant de produire et/ou d'introduire des radicaux hydroxyle (OH·) dans l'eau de procédé (3) pendant le fonctionnement de la machine de traitement de récipients (1) est réalisée de telle manière que des impuretés organiques (OV) présentes dans ladite machine de traitement de récipients sont minéralisées en impuretés inorganiques grâce aux radicaux hydroxyle (OH') fournis dans l'eau de procédé (3) afin de les éliminer de la machine de traitement de récipients (1) par lavage.

10. Machine de traitement de récipients selon la revendication 9, dans laquelle l'unité de préparation (9, 15) est réalisée en vue de la production non photochimique et/ou de l'introduction de radicaux hydroxyle (OH') au niveau d'une cuve de collecte (8) formée dans la machine de traitement de récipients (1), en particulier dans le pasteurisateur tunnel, et/ou en vue de la production photochimique de radicaux hydroxyle (OH') dans un circuit de préparation (6) présent au niveau de la machine de traitement de récipients (1), en particulier au niveau du pasteurisateur tunnel, et destiné à l'eau de procédé (3).

11. Machine de traitement de récipients selon la revendication 9 ou 10, comprenant en outre au moins une lampe UV (10) permettant d'irradier l'eau de procédé (3) et un dispositif de dosage (12) formé dans une région d'alimentation (14) menant à la lampe UV (10) et destiné à un agent d'oxydation photoréactif (13).

12. Machine de traitement de récipients selon la revendication 11, dans laquelle le dispositif de dosage (12) est agencé à une distance de pilotage d'au plus 1 m devant la lampe UV (10).

13. Machine de traitement de récipients selon la revendication 11 ou 12, dans laquelle la lampe UV (10) fait partie d'une unité de préparation (9) permettant de désinfecter l'eau de procédé (3) par UV.

14. Machine de traitement de récipients selon l'une quelconque des revendications 9 à 13, dans laquelle l'unité de préparation (15) comprend un dispositif de dosage (16) qui est agencé dans la région d'une cuve de collecte (8) destinée à l'eau de procédé (3) et qui permet d'ajouter une solution de minéralisation (17) contenant des radicaux hydroxyle (OH') à l'eau de procédé (3).
